# EUROPEAN PATENT SPECIFICATION

(11) **EP 3 317 671 B1**
(45) Date of publication and mention of the grant of the patent: **13.11.2019**
(21) Application number: 16736594.9
(22) Date of filing: 01.07.2016
(51) Int. Cl.: G01N 33/574, G01N 33/68, C12Q 1/68

(54) **USE OF CELL-FREE NUCLEOSOMES AS BIOMARKERS IN SPUTUM SAMPLES**
VERWENDUNG VON ZELLFREIEN NUKLEOSOMEN ALS BIOMARKER IN SPEICHELPROBEN
UTILISATION DE NUCLÉOSOMES ACELLULAIRES COMME BIOMARQUEURS DANS DES ÉCHANTILLONS D'EXPETCTORATION

(30) Priority: 01.07.2015 GB 201511512
(43) Date of publication of application: 09.05.2018
(73) Proprietor: Belgian Volition SPRL, 5032 Isnes (BE)
(72) Inventor: MICALLEF, Jacob, BE-5032 Isnes (BE); HERZOG, Marielle, BE-5032 Isnes (BE)
(74) Representative: Aitken, Elizabeth Grace Catherine
(86) International application number: PCT/GB2016/051994
(87) International publication number: WO 2017/001863

(56) References cited:
- WO-A1-2013/030577
- WO-A1-2013/030579
- WO-A1-2014/131841
- WO-A2-2013/084002
- GIULIA DE MAIO: "Circulating and stool nucleic acid analysis for colorectal cancer diagnosis", WORLD JOURNAL OF GASTROENTEROLOGY, vol. 20, no. 4, 28 January 2014 (2014-01-28), page 957, XP55299064, CN ISSN: 1007-9327, DOI: 10.3748/wjg.v20.i4.957
- VAN DER DRIFT M A ET AL: "Can free DNA be detected in sputum of lung cancer patients?", LUNG CANCER, ELSEVIER, AMSTERDAM, NL, vol. 61, no. 3, 1 September 2008 (2008-09-01), pages 385-390, XP025347223, ISSN: 0169-5002, DOI: 10.1016/J.LUNGCAN.2008.01.007 [retrieved on 2008-03-04]
- Anonymous: "Aberrant Promoter Methylation in Bronchial Epithelium and Sputum from Current and Former Smokers | Cancer Research", , 15 April 2002 (2002-04-15), XP055299414, Retrieved from the Internet: URL:http://cancerres.aacrjournals.org/cont ent/62/8/2370.long [retrieved on 2016-09-01]
- PRINSEN CLEMENS F ET AL: "Naked DNA in sputum from patients with lung cancer and/or COPD.", PROCEEDINGS OF THE AMERICAN ASSOCIATION FOR CANCER RESEARCH ANNUAL MEETING, vol. 45, March 2004 (2004-03), page 1091, XP002761388, & 95TH ANNUAL MEETING OF THE AMERICAN-ASSOCIATION-FOR-CANCER-RESEARCH; ORLANDO, FL, USA; MARCH 27 -31, 2004 ISSN: 0197-016X

## Description

### FIELD OF THE INVENTION

The invention relates to the use of cell-free nucleosomes, and in particular an epigenetic feature of cell-free nucleosomes as a biomarker for disease. The invention also provides a method for the detection of lung cancer and pre-cancer by detecting and measuring the presence of nucleosomes and epigenetically altered nucleosomes in sputum samples. In particular the method relates to the detection and epigenetic profiling of nucleosome chromatin fragments in sputum samples and using this epigenetic information to establish their cellular origination in the chromatin of healthy lung cells or in the chromatin of lung cancer cells.

### BACKGROUND OF THE INVENTION

Lung cancer is a common disease with an estimated 1.8 million new cases and 1.6 million deaths worldwide in 2012. The majority of lung cancer cases are associated with smoking. Mortality varies greatly depending on whether the disease is detected at an early localized stage, when effective treatment options are available, or at a late stage when the disease may have spread within the lung or beyond when treatment is more difficult. The 5-year survival rate may be as high as 80% for those in whom the disease is detected at stage IA, but only about 4% for those in whom late stage disease is detected. Unfortunately most cancers are currently detected at late stage.

Safe, non-invasive methods for the detection of lung cancer would enable early stage cancer detection, leading to earlier treatment and many years of saved life. Earlier cancer detection would also save money and resources for healthcare providers by reducing the need for expensive late stage cancer drug therapies and hospitalizations. Ideally lung cancer tests would also identify potentially malignant or precancerous nodules of the lung which may progress to cancer if left untreated, but can be removed or monitored if identified.

Current methods for lung cancer detection include chest X-rays and CT scans but these methods have poor clinical accuracy and subject patients to potentially dangerous doses of X-rays. Studies have demonstrated that screening with low-dose CT scanning detects potentially cancerous nodules in high-risk populations and decreases mortality. However, low dose CT scanning also has drawbacks including its subjective interpretation, a low clinical specificity for lung cancer leading to a high false positive rate and potential safety issues arising from repeated use of X-rays even at lower dose. The low clinical specificity of X-rays and CT scanning methods means they detect nodules of unknown etiology in the lungs of many subjects which cannot be identified as malignant or non-malignant in nature and this has led to another diagnostic challenge for oncologists. There is therefore a clinical need for a non-invasive test for the differential diagnosis of malignant, from non-malignant, nodules of the lung.

Other lung cancer test methods under research include DNA sequence mutation tests and specific gene sequence methylation tests. DNA abnormalities are characteristic of all cancer diseases. The DNA of cancer cells differs from that of healthy cells in many ways including, but not limited to, point mutations, translocations, gene copy number, micro-satellite abnormalities, DNA strand integrity and nucleotide modifications (for example methylation of cytosine at position 5). These tumor associated alterations in DNA structure or sequence are investigated routinely in cancer cells or tissue removed at biopsy or surgery for clinical diagnostic, prognostic and personalized treatment selection purposes. However, tissue DNA tests are too invasive for widespread testing or screening purposes.

The blood of cancer patients contains circulating tumor DNA (ctDNA) which is thought to originate from dying or dead cancer cells. Investigation of matched blood and tissue samples from cancer patients shows that cancer associated mutations, present in a patient's tumor (but not in his/her healthy cells) are also present in ctDNA in blood samples taken from the same patient (Newman *et al,* 2014). Similarly DNA sequences, particularly gene promoter sequences, that are differentially methylated (epigenetically altered by methylation of cytosine residues) in cancer cells can also be detected as methylated sequences in ctDNA in the circulation.

One challenge for ctDNA sequence mutation tests is that any particular sequence mutation will only occur in a minority (usually a small minority) of cancers. Therefore any cancer detection method relying on detection of a sequence mutation will have low clinical sensitivity and detect only a small proportion of cancers even if it has perfect analytical performance and always detects the mutation if present. For example, where a particular mutation occurs in only 5% of cancers then any such test can only have a clinical sensitivity of 5% even if the method itself has an analytical sensitivity of 100%. However, because the DNA of all cancers does contain sequence mutations; the clinical sensitivity for cancer detection can be increased by testing for multiple DNA sequences which are commonly mutated in cancer. In a recent lung cancer study, blood samples taken from lung cancer patients were tested for 534 frequently mutated genetic sequences from 139 known oncogenes using deep sequencing methods. Mutations were found in samples from all patients with stage II, III & IV cancers and in 50% of patients with stage I cancers (Newman *et al*, 2014). The cost of deep sequencing for hundreds of mutations makes this technique unsuitable for routine clinical use but the method may form a basis for future clinical tests.

Hypermethylation of certain specific ctDNA gene sequences has also been investigated for potential biomarker utility in lung cancer. For example, detection of hypermethylated Septin-9 gene sequences by amplification of DNA extracted from plasma was reported to detect 44% of lung cancers (Powrozek *et al,* 2014). The DNA methylation status of specific genes or loci is usually detected by selective bisulphite deamination of cytosine, but not 5-methylcytosine (5mc), to uracil, leading to a primary DNA sequence change that can be detected by sequencing or other means (Allen *et al,* 2004).

There is a clinical need for accurate, safe, non-invasive, low cost tests to identify individuals with early stage lung cancer, especially in smokers. Unfortunately, no such tests are currently available and only about 20% of lung cancers are detected early.

The present invention relates to the detection of cell-free nucleosomes and epigenetic features of cell free nucleosomes in sputum samples. Cell free nucleosomes have previously been reported in the blood where they are thought to originate from dead or dying cells. The mechanism of release from dead cells into the circulation is not known but is thought to involve circulating macrophages that remove dead cells by phagocytosis (Jiang *et al,* 2003). The level of circulating nucleosomes found in the blood of healthy subjects is low, but elevated levels are often found in the blood of subjects with a variety of disease conditions including lung cancer (Holdenrieder, 2001). However, elevated blood nucleosome levels are a nonspecific marker of elevated levels of cell death and do not distinguish lung cancer from other cancers or from other diseases or from injuries or trauma. There are no reports of nucleosomes in the sputum.

Nucleosomes have an immense diversity of chemical structure due to their role in the epigenetic regulation of gene expression. The human body comprises many different cell types of widely different phenotypes and functions in the body despite all containing the same genome. This phenotypic diversity is due to the differential expression of the genome in different cell types. The control of differential gene expression is not entirely understood but the basic mechanisms include gene regulation by a number of interconnected epigenetic signals associated with the gene, including non-coding nucleic acid sequences as well as structural chromatin aspects including control of chromatin packing (for example; as euchromatin or heterochromatin), control of nucleosome positioning and nuclease accessible sites, chemical modification of DNA, for example methylation of cytosine residues, and variation in the composition and structure of the histone complex around which the DNA is wrapped, for example by post-translational modification of histone proteins or by inclusion of alternative histone isoforms. Thus the chemical structure of cellular chromatin, and its constituent nucleosomes, is subject to a great variety of epigenetic alterations which together form a complex gene regulatory system. This regulatory system is corrupted in cancer leading to global epigenomic changes in chromosome structure. The altered chromatin features lead to gene mis-regulation which is associated with cancer development and progression. Moreover, inhibition, prevention or reversal of these epigenetic feature changes to chromatin and nucleosomes using epigenetic drugs is effective in the treatment of cancer diseases. Such epigenetic drugs include, for example, Histone Deacetylase Complex inhibitor (HDACi), Histone Methyl Transferase inhibitor (HMTi) and DNA Methyl Transferase inhibitor (DNMTi) drugs.

The nucleosome is the basic repetitive unit of chromatin structure and consists of a protein complex of eight highly conserved core histones (comprising of a pair of each of the histones H2A, H2B, H3, and H4). Around this complex is wrapped approximately 146 base pairs of DNA. Another histone, H1 or H5, acts as a linker and is involved in chromatin compaction. The DNA is wound around consecutive nucleosomes in a structure often said to resemble "beads on a string" and this forms the basic structure of open or euchromatin. In compacted or heterochromatin this string is coiled and super coiled into a closed and complex structure (Herranz and Esteller, 2007).

The structure of nucleosomes in cellular chromatin can vary by Post Translational Modification (PTM) of histone proteins and by the inclusion of variant histone proteins. PTM of histone proteins occurs most commonly, but not only, on the tails of the eight core histones and common modifications include acetylation, methylation or ubiquitination of lysine residues as well as methylation of arginine residues and phosphorylation of serine residues. Histone modifications are known to be involved in epigenetic regulation of gene expression (Herranz and Esteller, 2007). The structure of the nucleosome can also vary by the inclusion of alternative histone isoforms or variants which are different gene or splice products and have different amino acid sequences. Histone variants can be classed into a number of families which are subdivided into individual types. The nucleotide sequences of a large number of histone variants are known and publicly available for example in the National Human Genome Research Institute NHGRI Histone DataBase (http://genome.nhgri.nih.gov/histones/complete.shtml), the GenBank (NIH genetic sequence) DataBase, the EMBL Nucleotide Sequence Database and the DNA Data Bank of Japan (DDBJ).

Histone variant and histone modification patterns present in healthy and diseased cells have been shown to differ in numerous (mostly immunohistochemical) studies (Herranz and Esteller, 2007). One disadvantage of immunohistochemical methods for clinical use in lung cancer detection is that tissue sample collection is invasive involving surgery or biopsy.

Nucleosome structure also varies by chemical modification of their DNA component, including DNA methylation (Herranz and Esteller, 2007). It has been known in the art for some time that DNA may be methylated at the 5 position of cytosine nucleotides to form 5-methylcytosine and the involvement of DNA methylation in cancer was reported as early as 1983 (Feinberg and Vogelstein, 1983). DNA methylation patterns observed in cancer cells differ from those of healthy cells. Repetitive elements, particularly around pericentromeric areas, are reported to be hypomethylated in cancer relative to healthy cells but promoters of specific genes are reported to be hypermethylated in cancer. The balance of these two effects is reported to result in global DNA hypomethylation in cancer and this pattern is a hallmark of cancer cells (Esteller 2007, Hervouet *et al,* 2010, Rodriguez-Paredes & Esteller, 2011).

Nucleosome structure also varies by nucleosome binding to any of a multitude of other proteins present in chromatin to form nucleosome adducts. Chromatin comprises a large number of non-histone proteins of a wide variety of types with a variety of functions including transcription factors, transcription enhancement factors, transcription repression factors, histone modifying enzymes, DNA damage repair proteins, nuclear hormone receptors and many more, bound to its constituent DNA and/or histones (Yoshida and Shimura, 1972). The study of chromatin bound proteins has been carried out largely by Chromatin ImmunoPrecipitation (ChIP) methods. These methods are well known in the art but are complex, laborious and expensive.

Cell free nucleosomes perse can be detected by Enzyme-Linked ImmunoSorbant Assay (ELISA) and several methods have been reported (Salgame *et al,* 1997; Holdenrieder *et al,* 2001; van Nieuwenhuijze *et al,* 2003). These assays typically employ an anti-histone antibody (for example anti-H2B, anti-H3 or anti-H1, H2A, H2B, H3 and H4) as capture antibody and an anti-DNA or anti-H2A-H2B-DNA complex antibody as detection antibody.

We have previously reported ELISA assays for nucleosomes containing particular epigenetic signals including nucleosomes containing particular histone modifications, particular histone variants and particular DNA modifications as well as particular nucleosome adducts (WO 2005/019826, WO 2013/030579, WO 2013/030577, WO 2013/084002). We have previously used these assays to show that epigenetically altered circulating cell free nucleosomes can be detected in the blood of diseased patients.

Surprisingly we have now demonstrated the detection of cell free nucleosome chromatin fragments in sputum samples obtained from patients and have shown that the epigenetic nature or structural profile of these nucleosomes can be used to determine their origination. Chromatin fragments have been identified as originating from healthy cells or from diseased cells including from lung cancer cells or from a mixture of cells. The present invention involves no DNA sequencing of particular genes but relies on measurement of sputum nucleosome levels *per se* as well as the investigation of genome wide epigenetic alteration of nucleosome or other chromatin fragments in the sputum. We now report simple immunoassay methods for the direct estimation of nucleosomes and epigenetically altered cell free nucleosomes and nucleosome adducts in sputum samples and their determination as having a healthy or cancer cell chromatin origin.

### SUMMARY OF THE INVENTION

According to a first aspect of the invention there is provided the use of a cell free nucleosome as a biomarker in a sputum sample, for the diagnosis or detection of lung cancer.

According to a second aspect of the invention there is provided a method for diagnosing or detecting lung cancer in an animal or a human subject which comprises the steps of:
(ii) detecting or measuring an epigenetic feature of a cell-free nucleosome in a sputum sample obtained from the subject; and
(iii) using the measured level of cell-free nucleosomes containing said epigenetic feature as indicative of the presence of said disease in the subject.

### BRIEF DESCRIPTION OF THE FIGURES

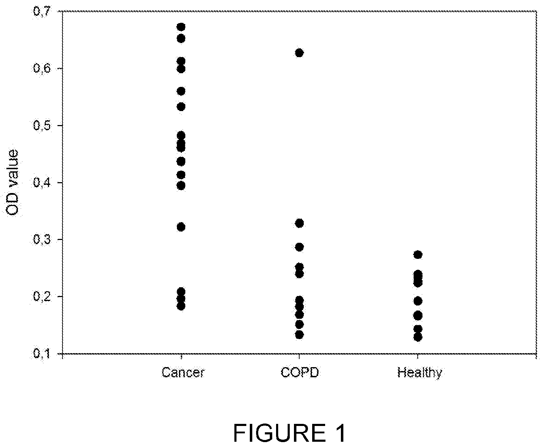

**Figure 1****:** Dot plot of the optical density results of a nucleosome associated H3K9Ac ELISA performed on sputum samples obtained from subjects with lung cancer, chronic obstructive pulmonary disorder (COPD) and approximately age-matched healthy control subjects.

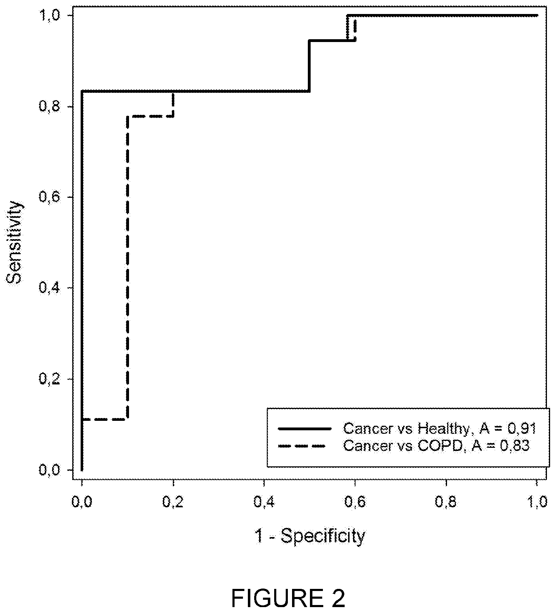

**Figure 2****:** ROC curve showing results obtained for the clinical accuracy of a nucleosome associated sputum H3K9Ac ELISA to distinguish subjects with lung cancer from approximately age-matched healthy control subjects and from subjects with chronic obstructive pulmonary disorder (COPD).

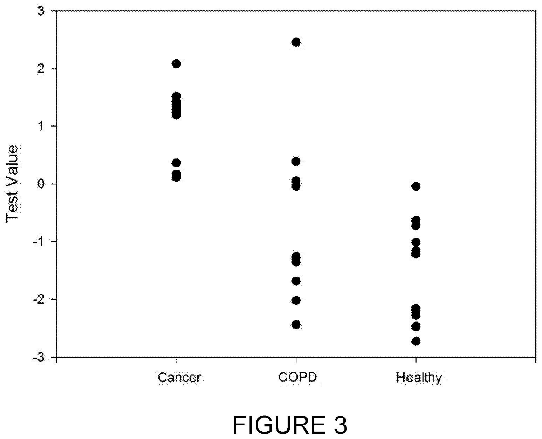

**Figure 3****:** Dot plot of the results of a panel test method of the invention including nucleosome associated H3K9Ac, H4K16Ac & H4K20Me3 performed on sputum samples obtained from subjects with lung cancer, chronic obstructive pulmonary disorder (COPD) and approximately age-matched healthy control subjects. Test values were calculated from the OD results of the individual assays using the arithmetic model; Test Value = 1.94[H3K9Ac] - 1.24[H4K16Ac] - 0.55[H4K20Me3]

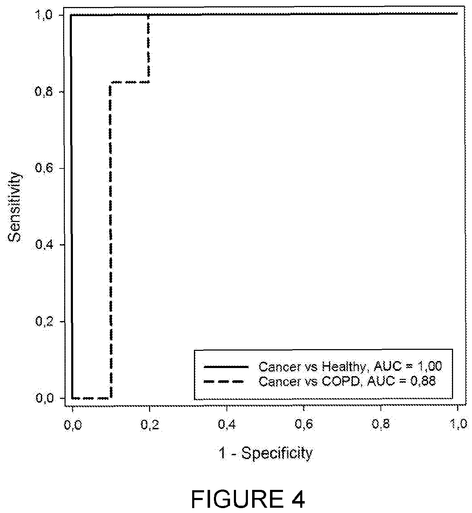

**Figure 4****:** ROC curve showing results obtained for the clinical accuracy of a panel test method of the invention including nucleosome associated H3K9Ac, H4K16Ac & H4K20Me3 to distinguish subjects with lung cancer from approximately age-matched healthy control subjects and from subjects with chronic obstructive pulmonary disorder (COPD).

### DETAILED DESCRIPTION OF THE INVENTION

The invention relates to the use of cell-free nucleosomes, and in particular an epigenetic feature of cell-free nucleosomes as a biomarker for lung cancer. The invention also provides a method for the detection of lung cancer and pre-cancer by detecting and measuring the presence of nucleosomes and epigenetically altered nucleosomes in sputum samples. In particular, the method relates to the detection and epigenetic profiling of nucleosome chromatin fragments in sputum samples and using this epigenetic information to establish their cellular origination in the chromatin of healthy lung cells or in the chromatin of lung cancer cells. We have previously reported the detection and epigenetic profiling of nucleosome chromatin fragments in blood samples (WO 2005/019826, WO 2013/030579, WO 2013/030577, WO 2013/084002). The presence of altered epigenetic chromatin fragments in a blood sample taken from a subject may be used to indicate the presence of a cancer in that subject, but may be less informative of the particular organ affected by that cancer because all tissues are perfused by blood. The particular advantage of the use of sputum samples is that the sample itself limits the origin of altered epigenetic chromatin fragments to the respiratory tract. The presence of altered epigenetic chromatin fragments in a sputum sample taken from a subject may be used to indicate the presence of a cancer in that subject and identify that cancer as a cancer of the lungs or respiratory tract.

According to a first aspect of the invention there is provided the use of a cell free nucleosome as a biomarker in a sputum sample, for the diagnosis or detection of lung cancer.

In one embodiment, the cancer includes pre-cancerous conditions and carcinoma-in-situ. We have shown that the level of nucleosomes (*per se*) is altered in the sputum samples of diseased patients relative to the levels of sputum samples of healthy subjects and that these nucleosomes comprise different absolute and relative levels of a variety of epigenetic structures. One advantage of sputum as a patient sample is the degree of organ specificity its use confers on biomarker tests.

In one embodiment, the biomarker is used for the diagnosis or detection of cancer, such as lung cancer, in particular non-small cell lung cancer (NSCLC).

In one embodiment, the cell-free nucleosome is a mononucleosome, oligonucleosome or other chromosome fragment.

In one embodiment, the sputum biomarker comprises the level of nucleosomes *per se.* It will be appreciated that the level of sputum nucleosomes *per se* may be estimated, for example using immunoassay methods for nucleosomes similar to those known in the art (Salgame *et al,* 1997; Holdenrieder *et al,* 2001; van Nieuwenhuijze *et al,* 2003).

In one embodiment, the sputum biomarker comprises an epigenetically altered or otherwise modified cell free nucleosome. Thus, for example, the biomarker is an epigenetic feature of a cell-free nucleosome.

According to a second aspect of the invention there is provided a method for diagnosing or detecting lung cancer in an animal or a human subject which comprises the steps of:
(ii) detecting or measuring an epigenetic feature of a cell-free nucleosome in a sputum sample obtained from the subject; and
(iii) using the measured level of cell-free nucleosomes containing said epigenetic feature as indicative of the presence of said disease in the subject.

In one embodiment, the epigenetic feature is selected from: a post-translational histone modification, a histone variant or isoform, a DNA modification or a protein adducted to the nucleosome

In a one embodiment, the epigenetic feature of a cell-free nucleosome comprises one or more histone post-translational modifications. A large number of such histone modifications are known in the art and this number is increasing with newly identified modifications. For example, without limitation, histones contain a variety of amino acid residues at multiple positions including lysine, serine, arginine and threonine residues in histones H2, H3 and H4, as well as their isoforms or sequence variants. Amino acid residues may be modified in multiple ways, for example without limitation, lysine residues may be acetylated, ubiquitinated, biotinylated or mono, di, or trimethylated. Any histone modification may be a suitable epigenetic feature for use in the invention whether detected or measured as an individual modified histone moiety, or whether detected or measured as a modified histone component of a cell free mononucleosome, oligonucleosome or other chromatin fragment, for example; using a chromatographic, mass spectrophotometric, biosensor, chromatin immunoprecipitation (ChIP), immunoassay or other method of detection. In a preferred embodiment nucleosome associated modified histones are measured by means of a 2-site immunoassay (immunometric) method utilising one antibody or other selective binder to a nucleosome epitope and another to the particular histone modification of interest. We have shown that the relative levels of nucleosome associated histone post-translational modification levels are altered in the sputum samples of diseased patients compared to the levels of sputum samples of healthy subjects.

In one embodiment of the invention a group or class of related histone modifications (rather than a single modification) is investigated in a sputum sample. A typical example of this embodiment, without limitation, would involve a 2-site immunoassay employing one antibody or other selective binder directed to bind to nucleosomes and one antibody or other selective binder directed to bind the group of histone modifications in question. Examples of such antibodies directed to bind to a group of histone modifications would include, for illustrative purposes without limitation, anti-pan-acetylation antibodies (eg; a Pan-acetyl H4 antibody), anti-citrullination antibodies or anti-ubiquitination antibodies.

In an alternative embodiment, the epigenetic feature of a cell-free nucleosome comprises one or more histone variants or isoforms. Many histone variants are known in the art (for example without limitation; variants of histone H2 include H2A1, H2A2, mH2A1, mH2A2, H2AX and H2AZ), and this number is increasing with newly identified isoforms. Histone isoform or variant protein structures are also amenable to post translational modification. For example; the H2A variant H2AX may be post translationally phosphorylated at serine 139 and this moiety is often called gamma-H2AX. Any histone variant, including any post translationally modified variant, may be a suitable epigenetic feature for use in the invention whether detected or measured as an individual histone variant moiety, for example using an immunoassay, chromatographic, mass spectrophotometric, ChIP, biosensor or other method for detection of a histone isoform moiety, or whether detected or measured as a histone component of a cell free mononucleosome, oligonucleosome or other chromatin fragment incorporating the histone variant. In a preferred embodiment nucleosome associated histone variants are measured by means of a 2-site immunoassay utilising one antibody or other selective binder to a nucleosome epitope and another to the particular histone variant or isoform of interest. We have shown that the relative levels of nucleosome associated histone variant levels are altered in the sputum samples of diseased patients compared to the levels of sputum samples of healthy subjects.

In an alternative embodiment, the epigenetic feature of a cell-free nucleosome comprises one or more DNA modifications. A number of particular DNA modifications are known in the art (for example; methylation, hydroxymethylation and carboxymethylation of cytosine) and this number is increasing with newly identified modifications. Any modified nucleotide may be a suitable epigenetic feature for use in the invention whether detected or measured in isolated DNA, nucleic acid, nucleotide or nucleoside moieties, for example using an immunoassay, chromatographic, mass spectrophotometric, ChIP, biosensor or other method for detection of a modified nucleic acid moiety, or whether detected or measured by any method for cell free mononucleosomes, oligonucleosomes or other chromatin fragments incorporating a particular modified nucleotide or DNA modification. In a preferred embodiment nucleosome associated DNA modifications are measured by means of a 2-site immunoassay utilising one antibody or other selective binder to a nucleosome epitope and another to the particular DNA modification of interest. We have shown that the relative levels of nucleosome associated 5-methylcytosine levels are altered in the sputum samples of diseased patients compared to the levels of sputum samples of healthy subjects. For clarity, the method of the present invention is not concerned with assessment of the methylation (or other DNA modification) status of any particular DNA sequence or gene, but with the global DNA modification status of the nucleosomes or chromatin fragments present in the sputum test samples.

In an alternative embodiment, the epigenetic feature of a cell-free nucleosome comprises one or more protein-nucleosome adducts or complexes. A large number of protein-nucleosome adducts or complexes are known to occur in chromatin (for example; HMGB, EZH2 and nuclear hormone receptor-nucleosome adducts) and this number is increasing with newly identified proteins that are active in chromatin. Any protein-nucleosome adduct may be a suitable epigenetic feature for use in the invention whether detected or measured by any method for cell free mononucleosome adducts, oligonucleosome adducts or other chromatin fragment adducts incorporating a particular protein, for example a 2-site immunoassay utilising one antibody or other selective binder to a nucleosome epitope and another to the particular protein comprised in the adduct. We have shown that the relative levels of nucleosome associated nucleosome adduct levels are altered in the sputum samples of diseased patients compared to the levels of sputum samples of healthy subjects. In one embodiment, two or more measurements of cell-free nucleosomes *per se* and/or cell-free nucleosome epigenetic features are performed as a panel of nucleosome features.

In a further embodiment, the biomarker comprises a panel of biomarkers selected from: the level of nucleosomes *per se,* one or more particular histone modifications or cell free nucleosomes comprising a particular histone modification, histone variants or cell free nucleosomes comprising a particular histone variant, DNA modifications or cell free nucleosomes comprising a particular DNA modification or nucleosome adducts. As different populations may not be epigenetically identical or similar, the optimum panels selected for different animals or different populations may vary. Thus a panel of such biomarkers selected for use to detect lung cancer (for example) in humans may not be the same as a panel selected for use in another species (for example cats or dogs). Similarly the optimal panel for use to detect lung cancer in male and female humans (or any other animal) may be different. Similarly the optimal panel for use to detect lung cancer in different human sub-populations or races may differ. Alternatively, the same panel may be used in different populations (for example in males and females) but with different weightings used for the panel assay members. For example; the test value of a three assay panel comprising assays "A", "B" and "C" may be calculated from a mathematical expression such as: "Test Value = A + 2B + 3C" in males with a cut-off value of "X" to determine a positive or negative result and from a different mathematical expression such as "Test Value = 3A + 2B + C" in females with a different cut-off value of "Y".

According to a further aspect of the invention, there is provided a method for detecting or quantifying an epigenetic feature of a cell-free nucleosome in a sputum sample obtained from an animal or a human subject which comprises the steps of:
(ii) contacting the sputum sample with a first binding agent which binds to cell-free nucleosomes or a component thereof;
(iii) contacting the sputum sample or cell-free nucleosomes with a second binding agent which binds to an epigenetic feature within said cell-free nucleosomes;
(iv) detecting or quantifying the binding of said second binding agent to the epigenetic feature within said cell-free nucleosomes in the sputum sample; and
(v) using the presence, degree or amount of such binding as a measure of the presence of cell-free nucleosomes containing said epigenetic feature in the sputum sample.

The epigenetic feature referred to may be a particular post-translational histone modification, a particular histone variant or isoform, a particular DNA modification or a particular protein adducted to said nucleosome.

According to a further aspect of the invention, there is provided a method for detecting or quantifying an epigenetic feature of a cell-free nucleosome in a sputum sample obtained from an animal or a human subject which comprises the steps of:
(ii) contacting the sputum sample with a first binding agent which binds to an epigenetic feature within said cell-free nucleosomes;
(iii) contacting the sputum sample or cell-free nucleosomes with a second binding agent which binds to cell-free nucleosomes or a component thereof;
(iv) detecting or quantifying the binding of said second binding agent to cell-free nucleosomes or a component thereof in the sputum sample; and
(v) using the presence, degree or amount of such binding as a measure of the presence of cell-free nucleosomes containing said epigenetic feature in the sputum sample.

The epigenetic feature referred to may be a particular post-translational histone modification, a particular histone variant or isoform, a particular DNA modification or a particular protein adducted to said nucleosome.

In a further aspect of the invention the measured level of nucleosomes containing a particular epigenetic structure is used to determine the cellular origin of nucleosomes present in a sputum sample. For example; whether sputum nucleosomes originate from the chromatin of healthy cells, lung cancer cells or cells with other lesions or from any mixture of such cells. It will be clear to those skilled in the art that a subject whose lungs contain cancer cells will also contain healthy cells and that a sputum sample obtained from such a subject may contain nucleosomes originating from healthy cells as well as from cancer cells. Similarly nucleosomes with other combinations of origins may occur in subjects with different medical conditions including nucleosomes from healthy cells as well as nucleosomes from one or more types of diseased cells with one or different lesions. In a preferred embodiment a panel of sputum nucleosome epigenetic structure levels is measured and the result is used to determine the cellular origin, or origins, of nucleosomes present in the sample.

According to a further aspect of the invention there is provided a method for determining the cell origin of sputum cell-free nucleosomes in an animal or a human subject which comprises the steps of:
(ii) detecting or measuring an epigenetic feature of a cell-free nucleosome in a sputum sample obtained from the subject; and
(iii) using the measured level of cell-free nucleosomes containing said epigenetic feature as an indicator of whether the cell-free nucleosomes originate from healthy lung cells, lung cancer cells or cells with other lesions or from any mixture of such cells.

In a preferred embodiment of the invention sputum assays for two or more epigenetic features are performed to produce an epigenetic profile consisting of multiple epigenetic nucleosome features. The epigenetic profile may be used to determine the origin of nucleosomes present in the sample (whether originating from the chromatin of healthy cells, lung cancer cells or cells with other lesions or from any mixture of such cells). The epigenetic profile may be used to determine the presence or otherwise of a lung cancer or other lesions in the subject from whom the sputum sample was taken.

According to a further aspect of the invention there is provided a method for detecting sputum cell-free nucleosomes with an epigenetic feature including nucleosome adducts and nucleosome associated histone modifications, DNA modifications and histone variants in a sputum sample obtained from a subject according to any of the above methods which comprises the steps of:
(ii) contacting the sputum sample with a first and second binding agent wherein one of said binding agents binds to cell-free nucleosomes or a component thereof and the other of said binding agents binds to an epigenetic feature of said cell-free nucleosomes;
(iii) detecting or quantifying the binding of either or both of said binding agents in the sputum sample; and
(iv) using the presence, degree or amount of such binding as a measure of the presence of cell-free nucleosomes with said epigenetic feature in the sputum sample.

It will be appreciated that the epigenetic features described herein may also be assayed independently of their inclusion, or otherwise, within a nucleosome. Thus, according to a further aspect of the invention there is provided a method for diagnosing or detecting lung cancer in an animal or a human subject which comprises the steps of:
(ii) detecting or measuring the level of a particular histone variant, histone isoform or a histone containing a particular post-translational modification or nucleosome adduct in the sputum sample;
(iii) using the measured level of the particular histone variant, isoform or modification in the sputum sample as an indicator of the presence of said disease in the subject.

In one embodiment of the invention histones in a sputum sample are assayed to determine the histone variant, isoform or histone modification composition of the said sample. In one embodiment the histones in a sputum sample may be isolated for such an assay, for example without limitation, by acid extraction of the sample. In another embodiment there is provided an immunoassay for a histone modification employing a first anti-histone binding agent in combination with another binding agent directed to bind a histone modification on the same histone moiety. In another embodiment there is provided a histone variant or isoform immunoassay employing two anti-histone binding agents directed to bind different epitopes on the same histone moiety at least one of which is specific to the isoform of interest.

According to a further aspect of the invention there is provided a method for diagnosing or detecting lung cancer in an animal or a human subject which comprises the steps of:
(ii) detecting or measuring the level of an epigenetically modified nucleotide in the sputum sample obtained from the subject and
(iii) using the measured level of the epigenetically modified nucleotide in the sputum sample as an indicator of the presence of said disease in the subject.

In a preferred embodiment of this aspect the epigenetically modified nucleotide measured in sputum chromatin fragments is 5-methylcytosine or methylated DNA. For clarity this aspect of the invention should not be confused with methods for gene methylation testing which involve the investigation of the cytosine methylation status of a particular gene or DNA sequence, for example as described by Powrozek *et al,* 2014. In contrast the present invention involves determination of the global level of 5-methylcytosine irrespective of gene sequence.

In one embodiment of the invention modified nucleotides in a sputum sample are assayed to determine the modified nucleotide composition of the said sample. In one embodiment the nucleotides and/or DNA in a sputum sample may be isolated for such an assay, for example without limitation, by organic solvent or chromatographic DNA extraction of the sample. In another embodiment there is provided an immunoassay for a modified nucleotide. This may be a single antibody immunoassay employing an anti-nucleotide binding agent, for example an anti-5methylcytosine binding agent, or a 2-site immunoassay, for example employing a first anti-single or double stranded DNA binding agent in combination with another binding agent directed to bind to a modified nucleotide.

In a preferred embodiment of the invention there is provided a 2-site immunoassay method for the measurement of nucleosome incorporated epigenetic features *in situ* employing an immobilized anti-nucleosome binding agent in combination with an anti-histone modification or anti-histone variant or anti-DNA modification or anti-adducted protein detection binding agent. In another embodiment of the invention there is provided a 2-site immunoassay employing an anti-nucleosome detection binding agent in combination with an immobilized anti-histone modification or anti-histone variant or anti-DNA modification or anti-adducted protein binding agent. In one embodiment, the nucleosome adduct includes a High Mobility Group Protein, a polycomb protein, a chromatin modifying enzyme or a nuclear hormone receptor.

In a preferred embodiment a panel of multiple epigenetic nucleosome features is used to detect the presence of a lung cancer in the subject. In addition, further tests may be included within such a panel to increase its clinical accuracy including for example tests for haemoglobin and/or other tumor markers (eg; carcinoembryonic antigen (CEA), CA125 and CA19.9) and/or specific mutated or methylated gene sequences. Additional parameters may include any relevant clinical information for example without limitation age, gender, Body Mass Index, smoking status and dietary habits.

In one embodiment of the invention the level of a particular structural epigenetic feature of a nucleosome in a sputum sample, or a panel of the levels of 2 or more such epigenetic features, is used to determine the optimal drug treatment or other treatment regime for a subject in need of such treatment.

According to a further aspect of the disclosure there is provided a method for assessment of an animal or a human subject for suitability for a medical treatment which comprises the steps of:
(i) detecting or measuring a cell-free nucleosome containing an epigenetic feature in a sputum sample obtained from the subject; and
(ii) using the measured level of cell-free nucleosomes with said feature detected as a parameter for selection of a suitable treatment for the subject.

According to a further aspect of the invention there is provided a method for monitoring the efficacy of a therapy in an animal or a human subject having, or suspected of having, or of being predisposed to lung cancer which comprises the steps of:
(ii) measuring an epigenetic feature of a cell-free nucleosome in the sputum sample obtained from the subject; and
(iii) using the measured level of cell-free nucleosomes containing said epigenetic feature compared with an earlier sample taken from said subject as indicative of the efficacy of said therapy.

According to a further aspect of the invention there is provided a method for determining the prognosis of an animal or a human subject with cancer, adenoma, autoimmune disease or inflammatory disease which comprises the steps of:
(ii) measuring an epigenetic feature of a cell-free nucleosome in the sputum sample obtained from the subject; and
(iii) using the level of cell-free nucleosomes containing said epigenetic feature as indicative of the prognosis of cancer, adenoma, autoimmune disease or inflammatory disease.

According to a further aspect of the invention there is provided a method for detecting or measuring sputum cell-free nucleosomes with an epigenetic feature, either alone or as part of a panel of measurements, for the purposes of detecting or diagnosing a disease status, or for assessment of an animal or a human subject for suitability for a medical treatment, or for monitoring a treatment of an animal or a human subject, for use in subjects with actual or suspected lung cancer.

According to a further aspect of the disclosure there is provided a method for identifying a sputum cell-free nucleosome associated epigenetic biomarker for detecting or diagnosing a disease status in an animal or a human subject which comprises the steps of:
(i) detecting or measuring a sputum cell-free nucleosome with an epigenetic feature in a sputum sample of a diseased subject;
(ii) detecting or measuring a cell-free nucleosome with the same epigenetic feature in a sputum sample of a healthy subject or a control subject; and
(iii) using the difference between the measured levels detected in a diseased and a control subject to identify whether a nucleosome with the epigenetic feature is useful as a biomarker for the disease status, either as a stand-alone biomarker or in combination with other biomarkers.

According to a further aspect of the invention there is provided the use of a kit comprising one or more binding agents capable of detecting and/or quantifying an epigenetic feature of a sputum cell-free nucleosome for the diagnosis or detection of lung cancer.

According to a further aspect of the invention a panel of tests is provided to obtain an epigenetic profile of sputum nucleosomes with regard to multiple epigenetic nucleosome features. Such a test panel may consist, for example, of two or more measurements of nucleosomes containing different nucleosome epitopes; including without limitation different adducts and/or histone variants and/or histone modifications and/or DNA modifications and/or measurements of nucleosomes *per se*, or any combination or ratio of any of these and any other nucleosome epitopes. It will be clear to those skilled in the art that any of these panels may also include other (non-epigenetic and/or non nucleosomal) markers including haemoglobin and any other known tumor markers.

It will be appreciated that any of the aforementioned methods may be used either as a stand-alone test or in combination with existing tests.

The epigenetic nucleosome profile obtained may be used to determine the cells of origin of the chromatin fragments in which the nucleosomes were produced. Likely cellular origins may include for example healthy cells, lung cancer cells, inflammation cells, COPD cells, asthmatic cells and cells of any other thoracic lesion. The sputum chromatin fragments may include fragments originating from a mixture of two or more different cell types, for example without limitation from healthy cells as well as lung cancer cells. If any (even a minority) of the nucleosome chromatin fragments present in the sputum sample originate from a diseased cell, for example a lung cancer cell, this indicates the presence of a disease condition, for example lung cancer, in the subject from whom the sputum was obtained.

It will be clear to those skilled in the art that the two antibodies or other binders used may be directed to bind to intact nucleosomes or to any component part of a nucleosome including without limitation against a histone, a histone variant, a histone modification or any nucleotide (modified or otherwise) or other part of the DNA component of a nucleosome. This applies in any combination to both the immobilised and detection antibodies (or other binders) used in a classical 2-site immunoassay. Thus in a further aspect of the invention there is provided a method for detecting (only) those nucleosomes which contain both features to which the binders are directed. An advantage of this design is that the sputum nucleosome epitopes can be selected to be epitopes whose concurrence in sputum nucleosomes is associated with healthy cells or lung cancer cells or otherwise diseased cells. Use of such an antibody or binder selective for an epitope common to many or most or all nucleosomes of a disease (eg; cancer) origin, but which is absent or rarer in nucleosomes of a healthy cell origin, as an immunosorbent (usually solid phase antibody) will select for such nucleosomes of diseased cell origin. This provides a tumor nucleosome selectivity or enrichment aspect to the assay.

In one embodiment of the invention, the antibody or other selective binder selected as the anti-nucleosome binder is selective for the enrichment of nucleosomes of tumor origin. In a preferred embodiment the antibody or other anti-nucleosome selective binder used is directed to bind to histone H3.1 and/or H3.2 and/or H3t. The binder selective for nucleosomes of tumor origin may be used in any assay including assays for the level of nucleosomes *per se* (of tumor origin), any particular histone modification or cell free nucleosomes comprising a particular histone modification, histone variant or cell free nucleosomes comprising a particular histone variant, DNA modification or cell free nucleosomes comprising a particular DNA modification or a nucleosome adduct.

Methods for the collection of sputum samples have been in use for many years and are well known in the art. The sputum collection method used in the present study involved asking subjects to breathe through a nebulizer containing NaCl solution to induce sputum formation for 5 minutes followed by coughing into a receptacle. If no sputum was produced this process was repeated up to a maximum of 3 further times (ie; up to a total of 20 minutes breathing on a nebulizer). The sample volume was measured and the sample was diluted with phosphate buffered saline. The diluted sample was centrifuged to remove cellular material, filtered through a gauze and frozen until used for assay.

Devices for the collection of sputum sample material are commercially available including, for example; the LungFlute which is a small self-powered audio device that induces sputum by generating sound waves and vibration in the airways of the lungs (Anjuman *et al,* 2013). It will be clear to those skilled in the art that any sputum sample collection method may be used for the invention. Similarly any sputum derived sample that has been diluted, filtered, separated, purified or otherwise prepared for analysis may be used be used for the invention.

Any method for isolating and/or detecting or measuring the level of histone modifications and/or cell free nucleosomes comprising a particular histone modification and/or histone variants and/or cell free nucleosomes comprising a particular histone variant and/or DNA modifications and/or cell free nucleosomes comprising a particular DNA modification and/or nucleosome adducts and/or nucleosomes *per se* as biomarkers in a sputum sample may be used for the invention. Examples of methods for the detection or measurement of theses analytes include chromatographic, spectroscopic methods (particularly mass spectrometry), biosensor, ChIP and immunochemical methods. Some detection or measurement methods may involve prior enrichment or isolation of these analytes from the sputum sample, for example by methods including solvent extraction (for example to extract nucleic acids), acid extraction (for example to extract histone proteins), chromatography or affinity purification/isolation using a selective antibody binder or other specific analyte binders.

We have demonstrated that methods of the invention can be used in sputum samples to detect non-small cell lung cancer (NSCLC), including early stage lung cancer. It will be clear to those skilled in the art that sputum matter may pass through or over a lung cancer. Breath condensate, bronchoalveolar lavage (BAL) and plural fluid are fluids which also pass through or over the thorax in which thoracic cancers and particularly lung cancers and pre-cancers may form. It is clear that methods of the invention may also be applied to breath condensate, BAL and plural fluid.

It will be clear to those skilled in the art that the methods of the invention described include a variety of embodiments including biosensor type assays and label-free assays of the type marketed for example by ForteBio Incorporated of USA.

It will be clear to those skilled in the art that the terms antibody, binder or ligand in regard to any aspect of the invention is not limiting but intended to include any binder capable of binding to particular molecules or entities and that any suitable binder can be used in the method of the invention. It will also be clear that the term nucleosomes is intended to include cell free mononucleosomes and oligonucleosomes and any such chromatin fragments that can be analysed in fluid media.

A further aspect of the disclosure provides ligands or binders, such as naturally occurring or chemically synthesised compounds, capable of specific binding to the biomarker. A ligand or binder according to the invention may comprise a peptide, an antibody or a fragment thereof, or a synthetic ligand such as a plastic antibody, or an aptamer or oligonucleotide, capable of specific binding to the biomarker. The antibody can be a monoclonal antibody or a fragment thereof capable of specific binding to the biomarker. A ligand according to the invention may be labeled with a detectable marker, such as a luminescent, fluorescent, enzyme or radioactive marker; alternatively or additionally a ligand according to the invention may be labelled with an affinity tag, e.g. a biotin, avidin, streptavidin or His (e.g. hexa-His) tag. Alternatively ligand binding may be determined using a label-free technology for example that of ForteBio Inc.

A biosensor according to the disclosure may comprise the biomarker or a structural/shape mimic thereof capable of specific binding to an antibody against the biomarker. Also provided is an array comprising a ligand or mimic as described herein.

Also provided by the disclosure is the use of one or more ligands as described herein, which may be naturally occurring or chemically synthesised, and is suitably a peptide, antibody or fragment thereof, aptamer or oligonucleotide, or the use of a biosensor of the invention, or an array of the invention, or a kit of the invention to detect and/or quantify the biomarker. In these uses, the detection and/or quantification can be performed on a biological sample as defined herein.

Diagnostic or monitoring kits are provided for performing methods of the invention. Such kits will suitably comprise a ligand according to the invention, for detection and/or quantification of the biomarker, and/or a biosensor, and/or an array as described herein, optionally together with instructions for use of the kit.

A further aspect of the disclosure is a kit for detecting the presence of a disease state, comprising a biosensor capable of detecting and/or quantifying one or more of the biomarkers as defined herein.

Biomarkers for detecting the presence of a disease are essential targets for discovery of novel targets and drug molecules that retard or halt progression of the disorder. As the level of the biomarker is indicative of disorder and of drug response, the biomarker is useful for identification of novel therapeutic compounds in *in vitro* and/or *in vivo* assays. Biomarkers of the invention can be employed in methods for screening for compounds that modulate the activity of the biomarker.

Thus, in a further aspect of the disclosure, there is provided the use of a binder or ligand, as described, which can be a peptide, antibody or fragment thereof or aptamer or oligonucleotide according to the invention; or the use of a biosensor according to the invention, or an array according to the invention; or a kit according to the invention, to identify a substance capable of promoting and/or of suppressing the generation of the biomarker.

Also there is provided a method of identifying a substance capable of promoting or suppressing the generation of the biomarker in a subject, comprising administering a test substance to a subject animal and detecting and/or quantifying the level of the biomarker present in a test sample from the subject.

The term "biomarker" means a distinctive biological or biologically derived indicator of a process, event, or condition. Biomarkers can be used in methods of diagnosis, e.g. clinical screening, and prognosis assessment and in monitoring the results of therapy, identifying subjects most likely to respond to a particular therapeutic treatment, drug screening and development. Biomarkers and uses thereof are valuable for identification of new drug treatments and for discovery of new targets for drug treatment.

The terms "detecting" and "diagnosing" as used herein encompass identification, confirmation, and/or characterisation of a disease state. Methods of detecting, monitoring and of diagnosis according to the invention are useful to confirm the existence of a disease, to monitor development of the disease by assessing onset and progression, or to assess amelioration or regression of the disease. Methods of detecting, monitoring and of diagnosis are also useful in methods for assessment of clinical screening, prognosis, choice of therapy, evaluation of therapeutic benefit, i.e. for drug screening and drug development.

Efficient diagnosis and monitoring methods provide very powerful "subject solutions" with the potential for improved prognosis, by establishing the correct diagnosis, allowing rapid identification of the most appropriate treatment (thus lessening unnecessary exposure to harmful drug side effects), and reducing relapse rates.

In one embodiment, said biomarker is released from the cells of a tumour. Thus, according to a further aspect of the invention there is provided a method for the detection of a tumour growth which comprises the steps of (i) measuring a biomarker in a biological sample that is associated with or released from the cells of a tumour and (ii) demonstrating that the level of said biomarker is associated with the size, stage, aggressiveness or dissemination of the tumour.

The immunoassays of the invention include any method employing one or more antibodies or other specific binders directed to bind to a nucleosome or to a nucleosome component or to an epigenetic feature of a nucleosome. Immunoassays include 2-site immunoassays or immunometric assays which may be label-less assays or employ (without limitation) radioactive, enzyme, fluorescent, time-resolved fluorescent, chemiluminescent, turbidimetric or particulate detection methods. Assays may also be single-site immunoassays, reagent limited immunoassays or competitive immunoassay methods including labelled antigen and labelled antibody single antibody immunoassay methods with a variety of label types including radioactive, enzyme, fluorescent, time-resolved fluorescent, chemiluminescent, turbidimetrc and particulate labels. All of said immunoassay methods are well known in the art.

In one embodiment, the method of the invention is repeated on multiple occasions. This embodiment provides the advantage of allowing the detection results to be monitored over a time period. Such an arrangement will provide the benefit of monitoring or assessing the efficacy of treatment of a disease state. Such monitoring methods of the invention can be used to monitor onset, progression, stabilisation, amelioration, relapse and/or remission.

Thus, the disclosure also provides a method of monitoring efficacy of a therapy for a disease state in a subject, suspected of having such a disease, comprising detecting and/or quantifying the biomarker present in a biological sample from said subject. In monitoring methods, test samples may be taken on two or more occasions. The method may further comprise comparing the level of the biomarker(s) present in the test sample with one or more control(s) and/or with one or more previous test sample(s) taken earlier from the same test subject, e.g. prior to commencement of therapy, and/or from the same test subject at an earlier stage of therapy. The method may comprise detecting a change in the nature or amount of the biomarker(s) in test samples taken on different occasions.

Thus, according to a further aspect of the disclosure there is provided a method for monitoring efficacy of therapy for a disease state in a human or animal subject, comprising:
(i) quantifying the amount of the biomarker as defined herein; and
(ii) comparing the amount of said biomarker in a test sample with the amount present in one or more control(s) and/or one or more previous test sample(s) taken at an earlier time from the same test subject.

A change in the level of the biomarker in the test sample relative to the level in a previous test sample taken earlier from the same test subject may be indicative of a beneficial effect, e.g. stabilisation or improvement, of said therapy on the disorder or suspected disorder. Furthermore, once treatment has been completed, the method of the invention may be periodically repeated in order to monitor for the recurrence of a disease.

Methods for monitoring efficacy of a therapy can be used to monitor the therapeutic effectiveness of existing therapies and new therapies in human subjects and in non-human animals (e.g. in animal models). These monitoring methods can be incorporated into screens for new drug substances and combinations of substances.

In a further embodiment the monitoring of more rapid changes due to fast acting therapies may be conducted at shorter intervals of hours or days.

According to a further aspect of the disclosure there is provided a method for identifying a biomarker for detecting the presence of a disease state. The term "identifying" as used herein means confirming the presence of the biomarker present in the biological sample. Quantifying the amount of the biomarker present in a sample may include determining the concentration of the biomarker present in the sample. Identifying and/or quantifying may be performed directly on the sample, or indirectly on an extract therefrom, or on a dilution thereof.

In alternative aspects of the invention, the presence of the biomarker is assessed by detecting and/or quantifying antibody or fragments thereof capable of specific binding to the biomarker that are generated by the subject's body in response to the biomarker and thus are present in a biological sample from a subject having a disease state.

Identifying and/or quantifying can be performed by any method suitable to identify the presence and/or amount of a specific protein in a biological sample from a subject or a purification or extract of a biological sample or a dilution thereof. In methods of the invention, quantifying may be performed by measuring the concentration of the biomarker in the sample or samples. Biological samples that may be tested in a method of the invention include those as defined hereinbefore. The samples can be prepared, for example where appropriate diluted or concentrated, and stored in the usual manner.

Identification and/or quantification of biomarkers may be performed by detection of the biomarker or of a fragment thereof, e.g. a fragment with C-terminal truncation, or with N-terminal truncation. Fragments are suitably greater than 4 amino acids in length, for example 5, 6, 7, 8, 9, 10, 11, 12, 13, 14, 15, 16, 17, 18, 19, or 20 amino acids in length. It is noted in particular that peptides of the same or related sequence to that of histone tails are particularly useful fragments of histone proteins.

The biomarker may be directly detected, e.g. by SELDI or MALDI-TOF. Alternatively, the biomarker may be detected directly or indirectly via interaction with a ligand or ligands such as an antibody or a biomarker-binding fragment thereof, or other peptide, or ligand, e.g. aptamer, or oligonucleotide, capable of specifically binding the biomarker. The ligand or binder may possess a detectable label, such as a luminescent, fluorescent or radioactive label, and/or an affinity tag.

For example, detecting and/or quantifying can be performed by one or more method(s) selected from the group consisting of: SELDI (-TOF), MALDI (-TOF), a 1-D gel-based analysis, a 2-D gel-based analysis, Mass spec (MS), reverse phase (RP) LC, size permeation (gel filtration), ion exchange, affinity, HPLC, UPLC and other LC or LC MS-based techniques. Appropriate LC MS techniques include ICAT® (Applied Biosystems, CA, USA), or iTRAQ® (Applied Biosystems, CA, USA). Liquid chromatography (e.g. high pressure liquid chromatography (HPLC) or low pressure liquid chromatography (LPLC)), thin-layer chromatography, NMR (nuclear magnetic resonance) spectroscopy could also be used.

Methods of diagnosing or monitoring according to the invention may comprise analysing a sample by SELDI TOF or MALDI TOF to detect the presence or level of the biomarker. These methods are also suitable for clinical screening, prognosis, monitoring the results of therapy, identifying subjects most likely to respond to a particular therapeutic treatment, for drug screening and development, and identification of new targets for drug treatment.

Identifying and/or quantifying the analyte biomarkers may be performed using an immunological method, involving an antibody, or a fragment thereof capable of specific binding to the biomarker. Suitable immunological methods include 2-site immunoassays (immunometric assays or sandwich immunoassays), such as sandwich ELISA, in which the detection of the analyte biomarkers is performed using two antibodies which recognize different epitopes on a analyte biomarker; radioimmunoassays (RIA), direct, indirect or competitive enzyme linked immunosorbent assays (ELISA), enzyme immunoassays (EIA), Fluorescence immunoassays (FIA), western blotting, immunoprecipitation and any particle-based immunoassay (e.g. using gold, silver, or latex particles, magnetic particles, or Q-dots). Immunological methods may be performed, for example, in microtitre plate or strip format.

According to a further aspect of the disclosure, there is provided a biomarker identified by the method as defined herein.

In one embodiment, one or more of the biomarkers may be replaced by a molecule, or a measurable fragment of the molecule, found upstream or downstream of the biomarker in a biological pathway.

According to a further aspect of the invention there is provided the use of a post-translational histone modification, a histone variant or isoform or a modified nucleotide as a biomarker in a sputum sample for the diagnosis or detection of lung cancer.

The identification of key biomarkers specific to a disease is central to integration of diagnostic procedures and therapeutic regimes. Using predictive biomarkers appropriate diagnostic tools such as biosensors can be developed; accordingly, in methods and uses of the invention, identifying and quantifying can be performed using a biosensor, microanalytical system, microengineered system, microseparation system, immunochromatography system or other suitable analytical devices. The biosensor may incorporate an immunological method for detection of the biomarker(s), electrical, thermal, magnetic, optical (e.g. hologram) or acoustic technologies. Using such biosensors, it is possible to detect the target biomarker(s) at the anticipated concentrations found in biological samples.

As used herein, the term "biosensor" means anything capable of detecting the presence of the biomarker. Examples of biosensors are described herein.

Biosensors according to the disclosure may comprise a ligand binder or ligands, as described herein, capable of specific binding to the biomarker. Such biosensors are useful in detecting and/or quantifying a biomarker of the invention.

The biomarker(s) of the invention can be detected using a biosensor incorporating technologies based on "smart" holograms, or high frequency acoustic systems, such systems are particularly amenable to "bar code" or array configurations.

In smart hologram sensors (Smart Holograms Ltd, Cambridge, UK), a holographic image is stored in a thin polymer film that is sensitised to react specifically with the biomarker. On exposure, the biomarker reacts with the polymer leading to an alteration in the image displayed by the hologram. The test result read-out can be a change in the optical brightness, image, colour and/or position of the image. For qualitative and semi-quantitative applications, a sensor hologram can be read by eye, thus removing the need for detection equipment. A simple colour sensor can be used to read the signal when quantitative measurements are required. Opacity or colour of the sample does not interfere with operation of the sensor. The format of the sensor allows multiplexing for simultaneous detection of several substances. Reversible and irreversible sensors can be designed to meet different requirements, and continuous monitoring of a particular biomarker of interest is feasible.

Suitably, biosensors for detection of one or more biomarkers of the invention combine biomolecular recognition with appropriate means to convert detection of the presence, or quantitation, of the biomarker in the sample into a signal. Biosensors can be adapted for "alternate site" diagnostic testing, e.g. in the ward, outsubjects' department, surgery, home, field and workplace.

Biosensors to detect one or more biomarkers of the invention include acoustic, plasmon resonance, holographic, Bio-Layer Interferometry (BLI) and microengineered sensors. Imprinted recognition elements, thin film transistor technology, magnetic acoustic resonator devices and other novel acousto-electrical systems may be employed in biosensors for detection of the one or more biomarkers of the invention.

Methods involving identification and/or quantification of one or more biomarkers of the invention can be performed on bench-top instruments, or can be incorporated onto disposable, diagnostic or monitoring platforms that can be used in a non-laboratory environment, e.g. in the physician's office or at the subject's bedside. Suitable biosensors for performing methods of the invention include "credit" cards with optical or acoustic readers. Biosensors can be configured to allow the data collected to be electronically transmitted to the physician for interpretation and thus can form the basis for e-medicine.

Diagnostic kits for the diagnosis and monitoring of the presence of a disease state are described herein. In one embodiment, the kits additionally contain a biosensor capable of identifying and/or quantifying a biomarker. Suitably a kit according to the invention may contain one or more components selected from the group: a ligand binder, or ligands, specific for the biomarker or a structural/shape mimic of the biomarker, one or more controls, one or more reagents and one or more consumables; optionally together with instructions for use of the kit in accordance with any of the methods defined herein.

The identification of biomarkers for a disease state permits integration of diagnostic procedures and therapeutic regimes. Detection of a biomarker of the invention can be used to screen subjects prior to their participation in clinical trials. The biomarkers provide the means to indicate therapeutic response, failure to respond, unfavourable side-effect profile, degree of medication compliance and achievement of adequate serum drug levels. The biomarkers may be used to provide warning of adverse drug response. Biomarkers are useful in development of personalized therapies, as assessment of response can be used to fine-tune dosage, minimise the number of prescribed medications, reduce the delay in attaining effective therapy and avoid adverse drug reactions. Thus by monitoring a biomarker of the invention, subject care can be tailored precisely to match the needs determined by the disorder and the pharmacogenomic profile of the subject, the biomarker can thus be used to titrate the optimal dose, predict a positive therapeutic response and identify those subjects at high risk of severe side effects.

Biomarker-based tests provide a first line assessment of 'new' subjects, and provide objective measures for accurate and rapid diagnosis, not achievable using the current measures.

Furthermore, diagnostic biomarker tests are useful to identify family members or subjects with mild or asymptomatic disease or who may be at high risk of developing symptomatic disease. This permits initiation of appropriate therapy, or preventive measures, e.g. managing risk factors. These approaches are recognised to improve outcome and may prevent overt onset of the disorder.

Biomarker monitoring methods, biosensors and kits are also vital as subject monitoring tools, to enable the physician to determine whether relapse is due to worsening of the disorder. If pharmacological treatment is assessed to be inadequate, then therapy can be reinstated or increased; a change in therapy can be given if appropriate. As the biomarkers are sensitive to the state of the disorder, they provide an indication of the impact of drug therapy.

According to a further aspect of the disclosure there is provided a method of treating cancer, adenoma, autoimmune disease or inflammatory disease in an animal or a human subject, which comprises the following steps:
(i) detecting or measuring an epigenetic feature of a cell-free nucleosome in the sputum sample of the subject;
(ii) using the measured level of cell-free nucleosomes containing said epigenetic feature as indicative of the presence of said disease in the subject; and
(iii) administering a therapeutic agent to a subject diagnosed in step (ii) as a patient with cancer, adenoma, autoimmune disease or inflammatory disease.

According to a further aspect of the disclosure there is provided a method of treating cancer, adenoma, autoimmune disease or inflammatory disease in an individual in need thereof, which comprises the step of administering a therapeutic agent to a patient identified as having differing levels of an epigenetic feature of a cell-free nucleosome in a sputum sample when compared to the levels of said epigenetic feature of a cell-free nucleosome from a control subject.

In one embodiment, the method of treatment comprises treatment of cancer, such as lung cancer, in particular non-small cell lung cancer (NSCLC).

The invention will now be illustrated with reference to the following non-limiting examples.

### EXAMPLE 1

Sputum samples were collected from 17 treatment naive subjects diagnosed with non-small cell lung cancer (NSCLC) including 2 subjects with stage T1 disease, 2 subjects with stage T2 disease, 5 subjects with stage T3 disease, 4 subjects with stage T4 disease and 4 subjects with disease of indeterminate stage Tx. Sputum samples were also collected from 10 subjects diagnosed with Chronic Obstructive Pulmonary Disorder (COPD) and 12 approximately aged match healthy control subjects. For collection, subjects were asked to breathe through a nebulizer containing NaCl solution to induce sputum formation for 5 minutes followed by coughing into a receptacle. If no sputum was produced this was repeated up to a maximum of 3 further times (i.e. up to a total of 20 minutes breathing on a nebulizer). The sample volume was measured and the sample was diluted 1+3 with phosphate buffered saline. The diluted sample was centrifuged to remove cellular material, filtered through a gauze and frozen until used for assay.

The diluted sputum samples were tested using an ELISA method of the invention for 9 different nucleosome assays including nucleosome concentration *per se*, as well as the following nucleosome associated epigenetic features; DNA modifications: 5-methylcytosine, Histone Variants: gamma-H2AX, Histone modifications: H3K9Me3, H3K9Ac, H3K27Me3, H4K16Ac, H4K20Me3, H4PanAc (pan-acetylatedH4). For each assay, 10µl of diluted sputum was added in duplicate to a microtitre well pre-coated with an anti-nucleosome antibody and incubated overnight at 4°C. The wells were washed three times with a wash buffer (200 µL/well, 0.05M Tris/HCI buffer pH 7.5 containing 1% Tween 20) and 50µl of a solution of biotinylated antibody (diluted in 0.05M Tris/HCI pH 7.5 containing 0.9% NaCl, 0.05% sodium deoxycholate and 1% Nonidet P40 substitute) directed to bind to the epigenetic feature of interest (i.e. directed to bind to nucleosomes *per se,* or 5-methylcytosine, or gamma-H2AX, or H3K9Me3, or H3K9Ac, or H3K27Me3, or H4K16Ac, or H4K20Me3 or H4PanAc respectively). The wells were incubated for a further 90 minutes at room temperature, washed again as before and horse radish peroxidase enzyme-labelled streptavidin solution was added and incubated for a further 30 minutes. The wells were washed again and a coloured substrate solution (100 µL/well, 2,2'-azinobis [3-ethylbenzothiazoline-6-sulfonic acid]-diammonium salt) was added and incubated for 20 minutes at room temperature. The optical density (OD) of the wells was measured at a wavelength of 405nm using a standard microtitre plate reader. A dose response curve of increasing colour with increasing nucleosome associated epigenetic structure concentration was observed with a low background signal observed in the negative control containing no nucleosomes.

The OD results of each of the 9 individual epigenetic nucleosome assays was plotted as a dot plot and as a Receiver Operator Characteristic (ROC) curve. The area under the curve (AUC) results are shown for all 9 assays in Table 1.

**Table 1**

| The discrimination of single ELISA methods of the invention for subjects with lung cancer from healthy subjects and for subjects with lung cancer from subjects with Chronic Obstructive Pulmonary Disorder (COPD). The results are expressed as percent Area Under the Curve (AUC) calculated by Receiver Operator Characteristic (ROC) analysis. | | |
|---|---|---|
| ***Single nucleosome epigenetic feature*** | ***% AUC Cancer vs healthy*** | ***% AUC Cancer vs COPD*** |
| Nucleosomes *per se* | 68 | 63 |
| 5-methylcytosine | 67 | 70 |
| Gamma-H2AX | 55 | 52 |
| H3K9Me3 | 63 | 57 |
| H3K9Ac | 91 | 83 |
| H3K27Me3 | 51 | 56 |
| H4K16Ac | 70 | 63 |
| H4K20Me3 | 57 | 52 |
| H4PanAc | 50 | 66 |

Table 1 shows that some of the epigenetic features tested are more altered in NSCLC than others. The most altered (highest AUC figures) were H3K9Ac, H4K16Ac, the nucleosome level *per se* and 5-methylcytosine. These assays were also generally the assays most different in cancer patients than COPD patients. A dot plot and ROC curve for the individual assay of sputum H3K9Ac are shown in Figures 1 and 2 respectively. Use of this single epigenetic feature of sputum chromosome fragments detected 82% of subjects with lung cancer with no false positive results for healthy subjects and surprisingly detected 78% of subjects with lung cancer with no false positive results for subjects with COPD. This demonstrates that the methods of the invention can detect lung cancer and distinguish it from other lung conditions. The ability to differentiate malignant and non-malignant lung conditions is an important unmet clinical need for lung cancer surgeons.

### EXAMPLE 2

The Optical Density (OD) results of the various single epigenetic feature measurements, made in EXAMPLE 1 above, were compiled in various combinations to obtain test panel results. The discrimination of multiple panel ELISA methods of the invention to identify subjects with lung cancer from healthy subjects and from subjects with Chronic Obstructive Pulmonary Disorder (COPD) was determined using simple arithmetic mathematical models calculated using Fisher's linear discriminant analysis of the OD results for the individual ELISA methods. The results were evaluated using ROC analysis of the respective models and the calculated clinical sensitivities for some panel methods, at a clinical specificity of 100% and at 90%, are shown in Table 2.

**Table 2**

| The discrimination of some multiple panel ELISA methods of the invention for subjects with lung cancer from healthy subjects and for subjects with cancer from subjects with Chronic Obstructive Pulmonary Disorder (COPD). Mathematical models for the panels were calculated using Fisher's linear discriminant analysis of the OD results for the individual ELISA methods. The results are expressed as observed percent clinical sensitivity at 100% or 90% specificity for NSCLC vs Healthy or for NSCLC vs COPD. | | |
|---|---|---|
| ***Nucleosome epigenetic feature panel*** | ***Cancer v Healthy Sensitivity at 100% (90%) specificity*** | ***Cancer v COPD Sensitivity at 100% (90%) specificity*** |
| H3K9Ac & H4K16Ac | 90% (100%) | 0% (82%) |
| H3K9Ac & H4K20Me3 | 100% (100%) | 0% (76%) |
| H3K9Ac & 5mc | 100% (100%) | 6% (6%) |
| H3K9Ac, 5mc & H4K16Ac | 100% (100%) | 6% (29%) |
| H3K9Ac, H4K16Ac & H4K20Me3 | 100% (100%) | 0% (82%) |

Clinical sensitivity values of 100%, with 100% specificity, showing perfect discrimination of cancer patients from healthy patients in this cohort were obtained for several assay panel combinations including H3K9Ac and 5-methylcytosine as shown in Table 2. Surprisingly, the combination of H3K9Ac and H4K20Me3 also gave perfect discrimination. This shows that an epigenetic feature which may be a poor discriminator of NSCLC alone (for example H4K20Me3) may none-the-less be useful as a discriminator when used as part of panel including other assays.

Surprisingly, the panel test results show 100% clinical accuracy (100% clinical sensitivity with 100% clinical specificity) for the discrimination of subjects with NSCLC from healthy subjects in this cohort for several different panel tests as well as excellent discrimination for cancer patients from COPD patients (Table 2). This demonstrates the accuracy and robustness of the invention. A dot plot and ROC curve obtained for the panel test including H3K9Ac, H4K16Ac and H4K20Me3 are shown in Figures 3 and 4. Use of this panel gave perfect discrimination of cancer patients from healthy subjects and a clinical sensitivity of 82% for the discrimination of subjects with NSCLC from subjects with COPD at 90% specificity. This is surprising because differential diagnosis of lung cancer from other lung conditions is a particularly difficult problem clinically. We conclude that these tests are safer than scanning methods for cancer detection that involve X-rays and the clinical accuracy achieved in this small subject cohort exceeds that of any non-invasive test for NSCLC currently available.

### EXAMPLE 3

A sputum sample is obtained from a lung cancer patient prior to a possible treatment regime with a drug or other therapy (for example an HDACi, HMTi, DNMTi or other epigenetic drug) and the epigenetic status of the tumor is assessed using a method of the invention (for example a 2-site immunoassay for nucleosomes containing a particular epigenetic feature or a panel of such 2-site immunoassays). The epigenetic status of sputum nucleosomes is used to determine which therapy is likely to be an effective or optimal treatment for the patient.

### EXAMPLE 4

A sputum sample is obtained from a lung cancer patient prior to a treatment regime (for example with an epigenetic drug including an HDACi, HMTi, DNMTi) and the epigenetic status of the tumor is assessed using a method of the invention (for example a 2-site immunoassay for nucleosomes containing a particular epigenetic feature or a panel of such 2-site immunoassays). Further sputum samples are obtained from the patient following treatment and the epigenetic status of sputum nucleosomes is reassessed for any change to check or monitor the patient for drug efficacy.

### EXAMPLE5

A sputum sample is obtained from a lung cancer patient and the epigenetic status of the tumor is assessed using a method of the invention (for example a 2-site immunoassay for nucleosomes containing a particular epigenetic feature or a panel of such 2-site immunoassays). The epigenetic status of sputum nucleosomes is used to assess the prognosis of for the patient.

### REFERENCES

Allen et al, A simple method for estimating global DNA methylation using bisulfite PCR of repetitive DNA elements. Nucleic Acids Research: 32(3) e38DOI: 10.1093/nar/gnh032
Anjuman et al. Evaluation of lung flute in sputum samples for molecular analysis of lung cancer. Clinical and Translational Medicine, 2, 15, 2013
Esteller, Cancer epigenomics: DNA methylomes and histone-modification maps Nature Reviews Genetics: 8, 286-298, 2007
Feinberg and Vogelstein, Hypomethylation distinguishes genes of some human cancers from their normal counterparts. Nature: 301, 89-92, 1983 doi: 10. 1371/journal.pone.0003759, 2008
Hervouet et al, Disruption of Dnmt1/PCNA/UHRF1 Interactions Promotes Tumorigenesis from Human and Mice Glial Cells
PLoS ONE 5(6): e11333. doi:10.1371/journal.pone.0011333, 2010
Herranz and Esteller, DNA methylation and histone modifications in patients with cancer: potential prognostic and therapeutic targets. Methods Mol Biol.361:25-62, 2007
Holdenrieder et al, Nucleosomes in serum of patients with benign and malignant diseases. Int. J. Cancer (Pred. Oncol.): 95, 114-120, 2001
Jiang et al, Blood. 102, 2243-50, 2003
The Medical Letter on Drugs and Therapeutics. 56, 100-101, 2014
Newman et al. An ultrasensitive method for quantitating circulating tumor DNA with broad patient coverage. Nature Medicine doi:10.1038/nm.3519, 2014
Powrozek et al. Septin 9 promoter region methylation in free circulating DNA - potential role in noninvasive diagnosis of lung cancer: preliminary report. Medical Oncology, 31, 917, 2014
Rodriguez-Paredes and Esteller, Cancer epigenetics reaches mainstream oncology. Nature Medicine: 17(3), 330-339, 2011
Salgame et al, An ELISA for detection of apoptosis. Nucleic Acids Research, 25(3), 680-681, 1997
van Nieuwenhuijze et al, Time between onset of apoptosis and release of nucleosomes from apoptotic cells: putative implications for systemic lupus erythematosus. Ann Rheum Dis; 62: 10-14, 2003
Yoshida and Shimura, Isolation of nonhistone chromosomal protein from calf thymus. Biochimica et Biophysica Acta (BBA) - Protein Structure; 263(3), 690-695, 1972

## Claims

1. Use of a cell free nucleosome as a biomarker in a sputum sample, for the diagnosis or detection of lung cancer.

2. Use as defined in claim 1, wherein the biomarker is an epigenetic feature of a cell-free nucleosome.

3. Use as defined in claim 1 or claim 2, wherein the cell-free nucleosome is a mononucleosome or oligonucleosome.

4. A method for diagnosing or detecting lung cancer in an animal or a human subject which comprises the steps of:
(i) detecting or measuring an epigenetic feature of a cell-free nucleosome in a sputum sample obtained from the subject; and
(ii) using the measured level of cell-free nucleosomes containing said epigenetic feature as indicative of the presence of said disease in the subject.

5. The use or method as defined in any one of claims 2 to 4, wherein the epigenetic feature is selected from: a post-translational histone modification, a histone variant or isoform, a DNA modification or a protein adducted to the nucleosome, such as a DNA modification selected from 5-methylcytosine, one or more histone variants selected from gamma-H2AX and H2AZ or one or more histone modifications selected from: H3K9Me3, H3K9Ac, H3K27Me3, H4K16Ac, H4K20Me3, ubiquityl-H2A and H4PanAc (pan-acetylatedH4).

6. A method for monitoring the efficacy of a therapy in an animal or a human subject having, or suspected of having, or of being predisposed to lung cancer which comprises the steps of:
(i) measuring an epigenetic feature of a cell-free nucleosome in a sputum sample obtained from the subject; and
(ii) using the measured level of cell-free nucleosomes containing said epigenetic feature compared with an earlier sample taken from said subject as indicative of the efficacy of said therapy.

7. A method for determining the prognosis of an animal or a human subject with lung cancer which comprises the steps of:
(i) measuring an epigenetic feature of a cell-free nucleosome in a sputum sample obtained from the subject; and
(ii) using the level of cell-free nucleosomes containing said epigenetic feature as indicative of the prognosis of lung cancer.

8. The use or method as defined in any one of claims 1 to 7, which is for the diagnosis or detection of non-small cell lung cancer (NSCLC).

9. A method for determining the cell origin of sputum cell-free nucleosomes in an animal or a human subject which comprises the steps of:
(i) detecting or measuring an epigenetic feature of a cell-free nucleosome in a sputum sample obtained from the subject; and
(ii) using the measured level of cell-free nucleosomes containing said epigenetic feature as an indicator of whether the cell-free nucleosomes originate from healthy lung cells, lung cancer cells or cells with other lesions or from any mixture of such cells.

10. The use or method as defined in any one of claims 1 to 9, wherein said detection or measurement comprises an immunoassay, immunochemical, mass spectroscopy, chromatographic, chromatin immunoprecipitation or biosensor method.

11. The use or method as defined in any one of claims 1 to 10, wherein two or more measurements of cell-free nucleosomes *per se* and/or cell-free nucleosome epigenetic features are performed as a panel of nucleosome features.

12. Use of a kit comprising one or more binding agents capable of detecting and/or quantifying an epigenetic feature of a sputum cell-free nucleosome for the diagnosis or detection of lung cancer in a sputum sample.

13. A method for detecting or quantifying an epigenetic feature of a cell-free nucleosome in a sputum sample obtained from an animal or a human subject which comprises the steps of:
(i) contacting the sputum sample with a first binding agent which binds to cell-free nucleosomes or a component thereof;
(ii) contacting the sputum sample or cell-free nucleosomes with a second binding agent which binds to an epigenetic feature within said cell-free nucleosomes;
(iii) detecting or quantifying the binding of said second binding agent to the epigenetic feature within said cell-free nucleosomes in the sputum sample; and
(iv) using the presence, degree or amount of such binding as a measure of the presence of cell-free nucleosomes containing said epigenetic feature in the sputum sample.

14. A method for detecting or quantifying an epigenetic feature of a cell-free nucleosome in a sputum sample obtained from an animal or a human subject which comprises the steps of:
(i) contacting the sputum sample with a first binding agent which binds to an epigenetic feature within said cell-free nucleosomes;
(ii) contacting the sputum sample or cell-free nucleosomes with a second binding agent which binds to cell-free nucleosomes or a component thereof;
(iii) detecting or quantifying the binding of said second binding agent to cell-free nucleosomes or a component thereof in the sputum sample; and
(iv) using the presence, degree or amount of such binding as a measure of the presence of cell-free nucleosomes containing said epigenetic feature in the sputum sample.

15. The method as defined in claim 13 or claim 14, wherein the epigenetic feature is selected from: a post-translational histone modification, a histone variant or isoform, a DNA modification or a protein adducted to said nucleosome, such as a DNA modifications selected from 5-methylcytosine, one or more histone variants selected from gamma-H2AX and H2AZ or one or more histone modifications selected from: H3K9Me3, H3K9Ac, H3K27Me3, H4K16Ac, H4K20Me3, ubiquityl-H2A and H4PanAc (pan-acetylatedH4).

## Patentansprüche

1. Verwendung eines zellfreien Nukleosoms als Biomarker in einer Speichelprobe zur Diagnose oder Detektion von Lungenkrebs.

2. Verwendung nach Anspruch 1, wobei der Biomarker ein epigenetisches Merkmal eines zellfreien Nukleosoms ist.

3. Verwendung nach Anspruch 1 oder Anspruch 2, wobei das zellfreie Nukleosom ein Mononukleosom oder ein Oligonukleosom ist.

4. Verfahren zum Diagnostizieren oder Detektieren von Lungenkrebs bei einem tierischen oder einem menschlichen Patienten, welches die folgenden Schritte umfasst:
(i) Detektieren oder Messen eines epigenetischen Merkmals eines zellfreien Nukleosoms in einer Speichelprobe, die von dem Patienten erhalten wurde; und
(ii) Verwenden des gemessenen Pegels von zellfreien Nukleosomen, die das epigenetische Merkmal enthalten, als Anzeichen für das Vorhandensein der Erkrankung bei dem Patienten.

5. Verwendung oder Verfahren nach einem der Ansprüche 2 bis 4, wobei das epigenetische Merkmal ausgewählt ist aus: einer posttranslationalen Histonmodifikation, einer Histonvariante oder -isoform, einer DNA-Modifikation oder einem Protein, welches an dem Nukleosom angelagert ist, wie etwa einer DNA-Modifikation, ausgewählt aus 5-Methylcytosin, einer oder mehreren Histonvarianten, ausgewählt aus gamma-H2AX und H2AZ oder einer oder mehreren Histonmodifikationen, ausgewählt aus: H3K9Me3, H3K9Ac, H3K27Me3, H4K16Ac, H4K20Me3, Ubiquityl-H2A und H4PanAc (pan-acetyliertem H4).

6. Verfahren zum Überwachen der Wirksamkeit einer Therapie bei einem tierischen oder einem menschlichen Patienten, der Lungenkrebs aufweist oder bei dem vermutet wird, dass er daran leidet, oder der dafür anfällig ist, welches die folgenden Schritte umfasst:
(i) Messen eines epigenetischen Merkmals eines zellfreien Nukleosoms in einer Speichelprobe, die von dem Patienten erhalten wurde; und
(ii) Verwenden des gemessenen Pegels von zellfreien Nukleosomen, die das epigenetische Merkmal enthalten, im Vergleich zu einer früheren Probe, die dem Patienten entnommen wurde, als Anzeichen für die Wirksamkeit der Therapie.

7. Verfahren zum Bestimmen der Prognose eines tierischen oder eines menschlichen Patienten mit Lungenkrebs, welches die folgenden Schritte umfasst:
(i) Messen eines epigenetischen Merkmals eines zellfreien Nukleosoms in einer Speichelprobe, die von dem Patienten erhalten wurde; und
(ii) Verwenden des Pegels von zellfreien Nukleosomen, die das epigenetische Merkmal enthalten, als Anzeichen für die Prognose von Lungenkrebs.

8. Verwendung oder Verfahren nach einem der Ansprüche 1 bis 7, die bzw. das zur Diagnose oder Detektion von nichtkleinzelligem Lungenkrebs (NSCLC) dient.

9. Verfahren zum Bestimmen des Zellursprungs von zellfreien Nukleosomen im Speichel bei einem tierischen oder einem menschlichen Patienten, welches die folgenden Schritte umfasst:
(i) Detektieren oder Messen eines epigenetischen Merkmals eines zellfreien Nukleosoms in einer Speichelprobe, die von dem Patienten erhalten wurde; und
(ii) Verwenden des gemessenen Pegels der zellfreien Nukleosome, die das epigenetische Merkmal enthalten, als Anzeichen dafür, ob die zellfreien Nukleosome aus gesunden Lungenzellen, Lungenkrebszellen oder Zellen mit anderen Läsionen oder aus einer Kombination solcher Zellen stammen.

10. Verwendung oder Verfahren nach einem der Ansprüche 1 bis 9, wobei die Detektion oder Messung ein Immunassay-, immunchemisches, Massenspektroskopie-, chromatographisches, Chromatin-Immunpräzipiations- oder Biosensor-Verfahren umfasst.

11. Verwendung oder Verfahren nach einem der Ansprüche 1 bis 10, wobei zwei oder mehr Messungen von zellfreien Nukleosomen an sich und/oder epigenetischen Merkmalen von zellfreien Nukleosomen als Gruppe von Nukleosom-Merkmalen durchgeführt werden.

12. Verwendung eines Sets, umfassend ein oder mehrere Bindemittel, die in der Lage sind, ein epigentisches Merkmal eines zellfreien Nukleosoms im Speichel zur Diagnose oder Detektion von Lungenkrebs in einer Speichelprobe zu detektieren und/oder zu quantifizieren.

13. Verfahren zum Detektieren oder Quantifizieren eines epigentischen Merkmals eines zellfreien Nukleosoms in einer Speichelprobe, die von einem tierischen oder einem menschlichen Patienten erhalten wurde, welches die folgenden Schritte umfasst:
(i) Kontaktieren der Speichelprobe mit einem ersten Bindemittel, welches an zellfreie Nukleosome oder eine Komponente davon bindet;
(ii) Kontaktieren der Speichelprobe oder der zellfreien Nukleosomen mit einem zweiten Bindemittel, welches an ein epigenetisches Merkmal in den zellfreien Nukleosomen bindet;
(iii) Detektieren oder Quantifizieren der Bindung des zweiten Bindemittels an das epigenetische Merkmal in den zellfreien Nukleosomen in der Speichelprobe; und
(iv) Verwenden des Vorhandenseins, des Ausmaßes oder der Menge eines solchen Bindemittels als ein Maß des Vorhandenseins der zellfreien Nukleosome, die das epigenetische Merkmal in der Speichelprobe enthalten.

14. Verfahren zum Detektieren oder Quantifizieren eines epigentischen Merkmals eines zellfreien Nukleosoms in einer Speichelprobe, die von einem tierischen oder einem menschlichen Patienten erhalten wurde, welches die folgenden Schritte umfasst:
(i) Kontaktieren der Speichelprobe mit einem ersten Bindemittel, welches an ein epigenetisches Merkmal in den zellfreien Nukleosomen bindet;
(ii) Kontaktieren der Speichelprobe oder der zellfreien Nukleosome mit einem zweiten Bindemittel, welches an zellfreie Nukleosome oder eine Komponente davon bindet;
(iii) Detektieren oder Quantifizieren der Bindung des zweiten Bindemittels an zellfreie Nukleosome oder eine Komponente davon in der Speichelprobe; und
(iv) Verwenden des Vorhandenseins, des Ausmaßes oder der Menge eines solchen Bindemittels als ein Maß des Vorhandenseins der zellfreien Nukleosome, die das epigenetische Merkmal in der Speichelprobe enthalten.

15. Verfahren nach Anspruch 13 oder Anspruch 14, wobei das epigenetische Merkmal ausgewählt ist aus: einer posttranslationalen Histonmodifikation, einer Histonvariante oder -isoform, einer DNA-Modifikation oder einem Protein, welches an dem Nukleosom angelagert ist, wie etwa DNA-Modifikationen, ausgewählt aus 5-Methylcytosin, einer oder mehreren Histonvarianten, ausgewählt aus gamma-H2AX und H2AZ oder einer oder mehreren Histonmodifikationen, ausgewählt aus: H3K9Me3, H3K9Ac, H3K27Me3, H4K16Ac, H4K20Me3, Ubiquityl-H2A und H4PanAc (pan-acetyliertem H4).

## Revendications

1. Utilisation d'un nucléosome acellulaire en tant que biomarqueur dans un échantillon d'expectoration, pour le diagnostic ou la détection d'un cancer du poumon.

2. Utilisation selon la revendication 1, dans laquelle le biomarqueur est une caractéristique épigénétique d'un nucléosome acellulaire.

3. Utilisation selon la revendication 1 ou la revendication 2, dans laquelle le nucléosome acellulaire est un mononucléosome ou un oligonucléosome.

4. Procédé pour diagnostiquer ou détecter un cancer du poumon chez un animal ou un sujet humain qui comprend les étapes consistant à :
(i) détecter ou mesurer une caractéristique épigénétique d'un nucléosome acellulaire dans un échantillon d'expectoration obtenu auprès du sujet ; et
(ii) utiliser le niveau mesuré de nucléosomes acellulaires contenant ladite caractéristique épigénétique comme indication de la présence de ladite maladie chez le sujet.

5. Utilisation ou procédé selon l'une quelconque des revendications 2 à 4, dans lequel la caractéristique épigénétique est sélectionnée à partir : d'une modification post-traductionnelle des histones, d'une variante ou d'une isoforme d'histone, d'une modification d'ADN ou d'une protéine ajoutée au nucléosome, telle qu'une modification d'ADN sélectionnée à partir de la 5-méthylcytosine, d'une ou plusieurs variantes d'histone sélectionnées à partir du gamma-H2AX et du H2AZ ou d'une ou plusieurs modifications des histones sélectionnées à partir de : H3K9Me3, H3K9Ac, H3K27Me3, H4K16Ac, H4K20Me3, ubiquityl-H2A et H4PanAc (H4 panacétylé).

6. Procédé pour surveiller l'efficacité d'une thérapie chez un animal ou un sujet humain ayant, ou soupçonné d'avoir, ou d'avoir une prédisposition à un cancer du poumon qui comprend les étapes consistant à :
(i) mesurer une caractéristique épigénétique d'un nucléosome acellulaire dans un échantillon d'expectoration obtenu auprès du sujet ; et
(ii) utiliser le niveau mesuré de nucléosomes acellulaires contenant ladite caractéristique épigénétique par rapport à un échantillon précédent prélevé auprès dudit sujet en tant qu'indication de l'efficacité de ladite thérapie.

7. Procédé pour déterminer le pronostic d'un animal ou d'un sujet humain atteint d'un cancer du poumon qui comprend les étapes consistant à :
(i) mesurer une caractéristique épigénétique d'un nucléosome acellulaire dans un échantillon d'expectoration obtenu auprès du sujet ; et
(ii) utiliser le niveau de nucléosomes acellulaires contenant ladite caractéristique épigénétique comme indication du pronostic d'un cancer du poumon.

8. Utilisation ou procédé selon l'une quelconque des revendications 1 à 7, qui est destiné au diagnostic ou à la détection d'un cancer du poumon non à petites cellules (CBNPC).

9. Procédé pour déterminer l'origine cellulaire de nucléosomes acellulaires d'expectoration chez un animal ou un sujet humain qui comprend les étapes consistant à :
(i) détecter ou mesurer une caractéristique épigénétique d'un nucléosome acellulaire dans un échantillon d'expectoration obtenu auprès du sujet ; et
(ii) utiliser le niveau mesuré de nucléosomes acellulaires contenant ladite caractéristique épigénétique en tant qu'indicateur du fait que les nucléosomes acellulaires sont ou ne sont pas originaires de cellules de poumon saines, de cellules de cancer du poumon ou de cellules atteintes d'autres lésions ou d'un quelconque mélange de telles cellules.

10. Utilisation ou procédé selon l'une quelconque des revendications 1 à 9, dans lesquels ladite détection ou mesure comprend un procédé utilisant un immunoessai, un essai immunochimique, une spectroscopie de masse, chromatographique, une immunoprécipitation de la chromatine ou des biocapteurs.

11. Utilisation ou procédé selon l'une quelconque des revendications 1 à 10, dans lesquels deux mesures ou plus de nucléosomes acellulaires en soi et/ou de caractéristiques épigénétiques de nucléosomes acellulaires sont réalisées en tant que panel de caractéristiques de nucléosomes.

12. Utilisation d'un kit comprenant un ou plusieurs agents de liaison capables de détecter et/ou de quantifier une caractéristique épigénétique d'un nucléosome acellulaire d'expectoration pour le diagnostic ou la détection d'un cancer du poumon dans un échantillon d'expectoration.

13. Procédé pour détecter ou quantifier une caractéristique épigénétique d'un nucléosome acellulaire dans un échantillon d'expectoration obtenu auprès d'un animal ou d'un sujet humain qui comprend les étapes consistant à :
(i) mettre en contact l'échantillon d'expectoration avec un premier agent de liaison qui se lie à des nucléosomes acellulaires ou à un composant de ceux-ci ;
(ii) mettre en contact l'échantillon d'expectoration ou les nucléosomes acellulaires avec un second agent de liaison qui se lie à une caractéristique épigénétique au sein desdits nucléosomes acellulaires ;
(iii) détecter ou quantifier la liaison dudit second agent de liaison à la caractéristique épigénétique au sein desdits nucléosomes acellulaires dans l'échantillon d'expectoration ; et
(iv) utiliser la présence, le degré ou la quantité d'une telle liaison en tant que mesure de la présence de nucléosomes acellulaires contenant ladite caractéristique épigénétique dans l'échantillon d'expectoration.

14. Procédé pour détecter ou quantifier une caractéristique épigénétique d'un nucléosome acellulaire dans un échantillon d'expectoration obtenu auprès d'un animal ou d'un sujet humain qui comprend les étapes consistant à :
(i) mettre en contact l'échantillon d'expectoration avec un premier agent de liaison qui se lie à une caractéristique épigénétique au sein desdits nucléosomes acellulaires ;
(ii) mettre en contact l'échantillon d'expectoration ou les nucléosomes acellulaires avec un second agent de liaison qui se lie à des nucléosomes acellulaires ou à un composant de ceux-ci ;
(iii) détecter ou quantifier la liaison dudit second agent de liaison à des nucléosomes acellulaires ou à un composant de ceux-ci dans l'échantillon d'expectoration ; et
(iv) utiliser la présence, le degré ou la quantité d'une telle liaison en tant que mesure de la présence de nucléosomes acellulaires contenant ladite caractéristique épigénétique dans l'échantillon d'expectoration.

15. Procédé selon la revendication 13 ou la revendication 14, dans lequel la caractéristique épigénétique est sélectionnée à partir : d'une modification post-traductionnelle des histones, d'une variante ou d'une isoforme d'histone, d'une modification d'ADN ou d'une protéine ajoutée audit nucléosome, telles que des modifications d'ADN sélectionnées à partir de la 5-méthylcytosine, d'une ou plusieurs variantes d'histone sélectionnées à partir du gamma-H2AX et du H2AZ ou d'une ou plusieurs modifications des histones sélectionnées à partir de :H3K9Me3, H3K9Ac, H3K27Me3, H4K16Ac, H4K20Me3, ubiquityl-H2A et H4PanAc (H4 panacétylé).
